# EUROPEAN PATENT SPECIFICATION

(11) **EP 4 338 716 B1**
(45) Date of publication and mention of the grant of the patent: **04.02.2026**
(21) Application number: 23170750.6
(22) Date of filing: 28.04.2023
(51) Int. Cl.: A61F 7/00, A61F 7/08, A61F 7/02

(54) **CONTROLLER FOR COOL AND WARM DUAL SYSTEM**
STEUERUNG FÜR EIN KÜHL- UND WÄRMEDOPPELSYSTEM
DISPOSITIF DE COMMANDE POUR SYSTÈME DOUBLE FROID ET CHAUD

(30) Priority: 15.09.2022 CN 202222447893 U
(43) Date of publication of application: 20.03.2024
(73) Proprietor: Shenzhen Youbosi Technology Co., Ltd., Shenzhen, Guangdong 518000 (CN)
(72) Inventor: Huang, Changwu, Shenzhen, 518000 (CN)
(74) Representative: Petraz, Gilberto Luigi

(56) References cited:
- WO-A1-2022/173547
- WO-A1-97/36560
- CN-U- 216 454 255
- US-A1- 2021 219 736

## Description

### CROSS-REFERENCE TO RELATED APPLICATIONS

The present application claims priority of Chinese Patent Application No. 202222447893.7, filed on Sep. 15, 2022.

### TECHNICAL FIELD

The present application relates to the technical field of cool and warm mattresses, and in particular to a controller for cool and warm dual system.

### BACKGROUND

The existing cool and warm dual system used in the mattress generally includes a controller, a mattress, and outlet and return pipes connected between the controller and the mattress. The controller controls the operation of the cool and warm dual system to achieve the dual effect of cold and warm. CN 216454255U provides a main machine for a cold and warm water circulation system, which includes a bottom shell and a top shell, a water tank and a water pump connected with the water tank are arranged in the bottom shell, a heater is arranged in the water tank, a water tank top cover is arranged on the water tank, and a heating sealing gasket is arranged between the water tank top cover and the heater. The two side walls, in a width direction of the bottom shell, of the water tank are each correspondingly provided with a containing cavity penetrating through the side wall of the water tank, the two containing cavities are each internally and correspondingly provided with a refrigeration end exchanger extending into the water tank, the outer sides of the two refrigeration end exchangers are each correspondingly provided with a refrigeration piece in an attached mode, and the outer sides of the two refrigeration pieces are each correspondingly provided with a radiator in an attached mode. The cooling end exchanger is provided with two radiators, the outer sides of the two radiators are respectively and correspondingly provided with a cooling fan in an attached mode, the outer sides of the two cooling fans are respectively and correspondingly provided with an air guide cover, and the two air guide covers are respectively arranged on the two radiators in a covering mode. However, the existing cool and warm dual system for the mattress needs to switch between the cooling process and heating process, which poses the problem of insufficient cooling and heating effects.

### SUMMARY

The invention is defined in appended independent claim 1, further embodiments are described in the dependent claims. The technical problem to be solved by the present application is to provide a controller for cool and warm dual system to solve the problem of insufficient cooling and heating effects in the existing cool and warm dual system for mattress.

The technical solution adopted by the present application to solve the above technical problem is as follows:

Provided is a controller for cool and warm dual system. The cool and warm dual system further includes a mattress and outlet and return pipes connected between the controller and the mattress. The controller controls the operation of the cool and warm dual system and includes a bottom housing and a top housing, a water tank and a water pump connected to the water tank being provided in the bottom housing, a heater being provided in the water tank, a tank cover being provided on the water tank, and a heating sealing gasket being sandwiched between the tank cover and the heater, accommodating slots being defined in and extending through two sidewalls of the water tank along its width direction, respectively, cooling end heat exchangers being respectively accommodated in the accommodating slots and extending into the water tank, outer sides of the cooling end heat exchangers being respectively provided with a cooling plate, and outer sides of the cooling plates being respectively provided with a semi-circular shaped heat sink, opposite sides of the two heat sinks respectively defining an inclined notch therein, and cooling fans matching with the notches being respectively arranged in the notches, the cooling fans being set obliquely and attaching to the heat sinks, respectively.

In some embodiments, a sidewall of the bottom housing along a length direction of the water tank is provided with two first fixing plates that are spaced from each other, two sidewalls of the bottom housing along the width direction of the water tank each are provided with a second fixing plate that corresponds to the first fixing plates and abuts one side of the water tank, and third fixing plates are provided between the two first fixing plates, correspond to the second fixing plates, and abut another side of the water tank.

In some embodiments, two sidewalls of the bottom housing along the width direction of the water tank each are provided with a fourth fixing plate that extends into the notch and abuts the heat sink.

In some embodiments, the cooling fan is set obliquely between corresponding second fixing plate and fourth fixing plate, with one side thereof abutting the second fixing plate and another side thereof abutting the fourth fixing plate.

In some embodiments, four sidewalls of the bottom housing define a plurality of first vents that extend vertically, and a bottom of the bottom housing is provided with two rows of second vents that are arranged in sequence and spaced from each other.

In some embodiments, a sidewall of the top housing neighboring to the two first fixing plates defines a plurality of third vents that extend transversely and correspond to the first vents.

In some embodiments, a water injection pipe is provided on the tank cover, a mounting port is provided on the top housing corresponding to the water injection pipe, and a pipe cover is provided on the mounting port.

According to the controller for cool and warm dual system provided by this application, which includes a bottom housing and the top housing, a water tank and a water pump connected to the water tank is provided in the bottom housing, a heater is provided in the water tank, a tank cover is arranged on the water tank, and a heating sealing gasket is sandwiched between the tank cover and the heater. Accommodating slots are defined in and extend through two sidewalls of the water tank along its width direction, respectively. The two accommodating slots each accommodate a heat exchanger that extends into the water tank. Outer sides of the two heat exchangers are respectively provided with a cooling plate, and outer sides of the two cooling plates are respectively provided with a semi-circular shaped heat sink. Opposite sides of the two heat sinks away from each other each define an inclined notch therein, and cooling fans matching with the notches are arranged in the two notches, respectively. The cooling fan is set obliquely, and attaches to the heat sink. In this way, by setting of the heat exchangers, the cooling plates, the heat sinks and the cooling fans, when the mattress needs to be heated, the heater is started to heat the water in the water tank, which is delivered to the mattress under the action of the water pump and through the outlet pipe. The water that flows through the mattress is circulated to the water tank through the return pipe, thereby achieving the effect of circulating heating the mattress; when the mattress needs to be cooled, the two cooling plates are activated to absorb heat from the water of the water tank through the two heat exchangers, respectively, thereby achieving cooling of the water in the water tank, which is delivered to the mattress under the action of the water pump and through the outlet pipe. The water that flows through the mattress is circulated to the water tank through the return pipe, thereby achieving the effect of circulating cooling the mattress. At the same time, heat of the two cooling plates is transferred to the two heat sinks, respectively, and then dissipated to the outside through the two cooling fans along a direction from one side adjacent to the notch towards another side away from the notch. Thus, the present application can achieve the dual control functions of cooling and heating.

### BRIEF DESCRIPTION OF DRAWINGS

In order to illustrate technical solutions of embodiments of the present application more clearly, drawings that need to be used in the description of the embodiments will be briefly described below. It is obvious that the drawings described below are only some embodiments of the present application, and other drawings may be obtained from the drawings without any creative work to those skilled in the art, which should be in the scope of this application.
FIG. 1 is a schematic, assembled view of a controller for cool and warm dual system according to an embodiment of the present application.
FIG. 2 is a side view of the controller for cool and warm dual system.
FIG. 3 is another side view of the controller for cool and warm dual system.
FIG. 4 is a bottom view of the controller for cool and warm dual system.
FIG. 5 is a schematic, exploded view of the controller for cool and warm dual system.
FIG. 6 is an exploded view of the controller for cool and warm dual system viewed from another aspect.
FIG. 7 is a schematic view of the controller for cool and warm dual system, wherein a top housing thereof is removed.
FIG. 8 is a schematic view of a bottom housing of the controller for cool and warm dual system.
FIG. 9 is a schematic view of a heat sink of the controller for cool and warm dual system.
FIG. 10 is another side view of the heat sink.

### DESCRIPTION OF EMBODIMENTS

For better illustrating the technical means, creative features, objects and effects of the present application, detailed description will be given for the embodiments provided by the present application with reference to the append drawings. Obviously, the described embodiments are only a part of the embodiments, and not all of the embodiments of the present application.

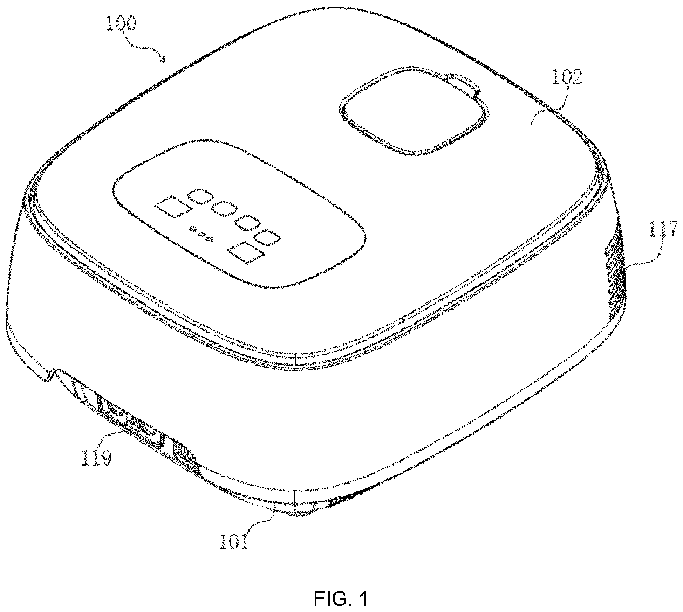

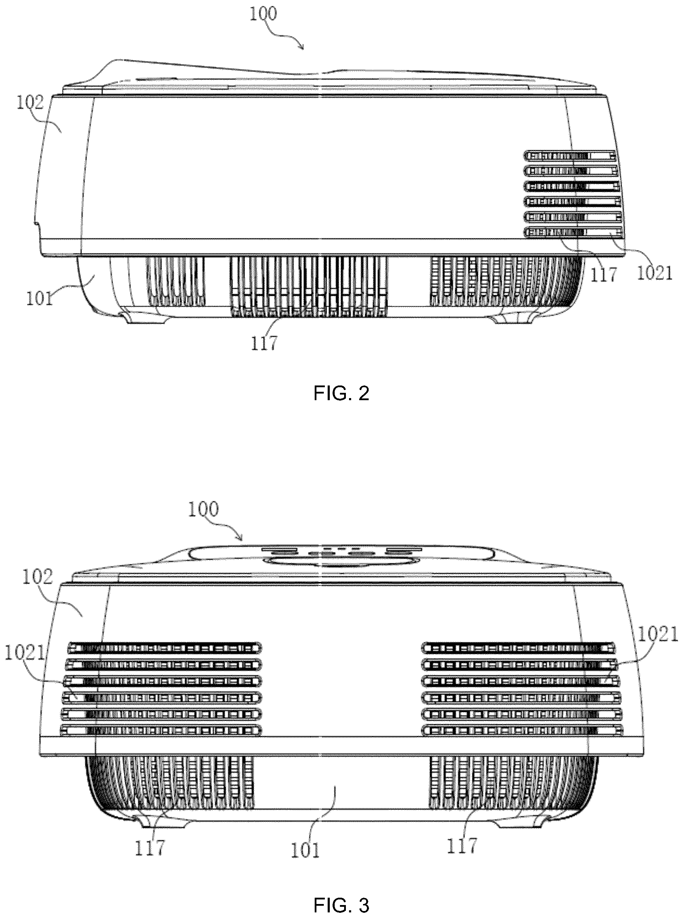

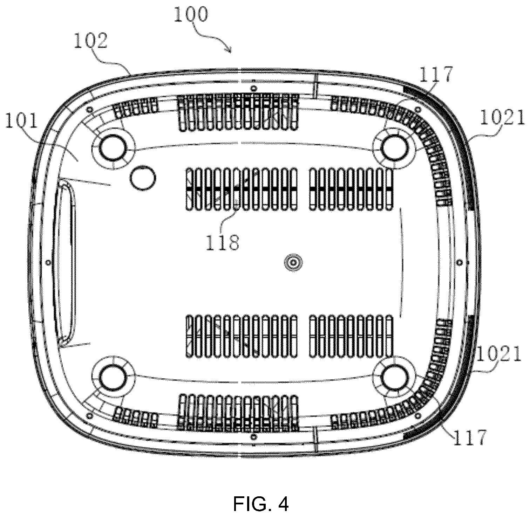

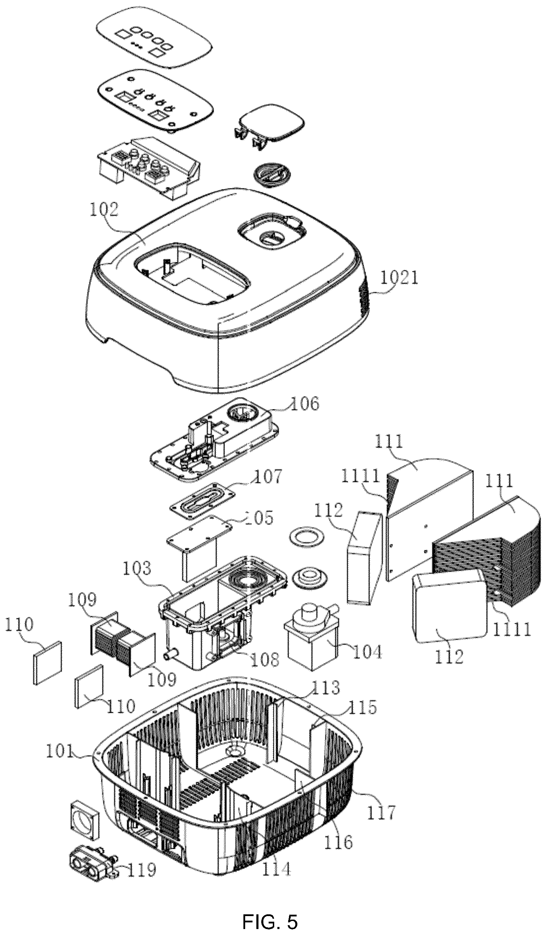

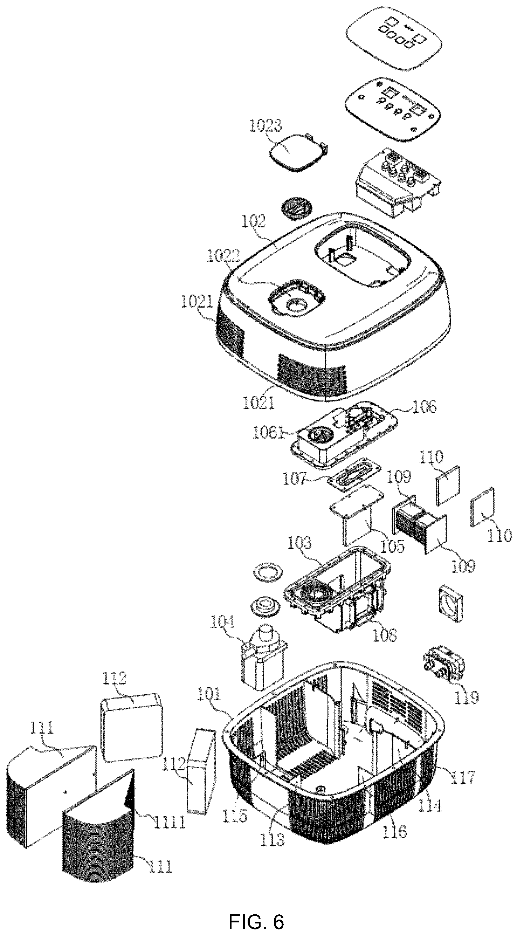

Please referring to FIG. 1 to FIG. 10, the present application provides a controller for cool and warm dual system 100. The cool and warm dual system further includes a mattress and outlet and return pipes connected between the controller 100 and the mattress. The controller 100 controls the operation of the cool and warm dual system, and includes a bottom housing 101 and the top housing 102. A water tank 103 and a water pump 104 connected to the water tank 103 is provided in the bottom housing 101, and a heater 105 is provided in the water tank 103. A tank cover 106 is arranged on the water tank 103, and a heating sealing gasket 107 is sandwiched between the tank cover 106 and the heater 105. The heating sealing gasket 107 has the function of sealing and insulating the heater 105.

Accommodating slots 108 are defined in and extend through two sidewalls of the water tank 103 along its width direction, respectively. The two accommodating slots 108 each accommodate a cooling end heat exchanger 109 that extends into an interior of the water tank 103. Outer sides of the two cooling end heat exchangers 109 are respectively provided with a cooling plate 110, and outer sides of the two cooling plates 110 are respectively provided with a semi-circular shaped heat sink111. Opposite sides of the two heat sinks 111 away from each other each define a notch 1111 therein, and each notch 1111 is set obliquely. Cooling fans 112 matching with the notches 1111 are arranged in the two notches 1111, respectively. The cooling fan 112 is set obliquely, and attaches to the heat sink 111.

In this way, by setting of the cooling end heat exchangers 109, the cooling plates 110, the heat sinks 111 and the cooling fans 112, opposite sides of the two heat sinks away from each other are provided with included notches 1111, and inclined cooling fans 112 are arranged in the notches 1112 and attach to the heat sinks 111, respectively. When the mattress needs to be heated, the heater 105 is started to heat the water in the water tank 103, which is delivered to the mattress under the action of the water pump 104 and through the outlet pipe. The water that flows through the mattress is circulated to the water tank 103 through the return pipe. The heating temperature may reach 22-70 degrees and the heating effect is well, thereby achieving the effect of circulating heating the mattress.

When the mattress needs to be cooled, the two cooling plates 110 are activated to absorb heat from the water of the water tank 103 through the two heat exchangers 109, respectively, thereby achieving cooling of the water in the water tank 103, which is delivered to the mattress under the action of the water pump 104 and through the outlet pipe. The water that flows through the mattress is circulated to the water tank 103 through the return pipe. The cooling temperature may reach 12-25 degrees and the cooling effect is well, thereby achieving the effect of circulating cooling the mattress. At the same time, heat of the two cooling plates 110 is transferred to the two heat sinks 111, respectively, and then dissipated to the outside through the two cooling fans 112 along a direction from one side adjacent to the notch 1111 towards another side away from the notch 1111. Thus, the present application can achieve the dual control functions of cooling and heating.

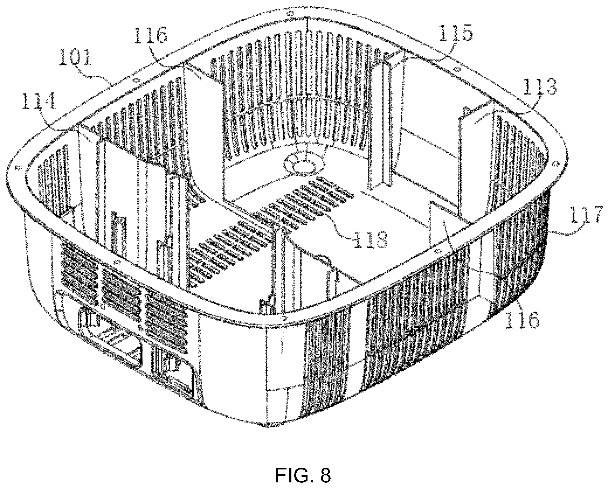

Referring to FIG. 8, a sidewall of the bottom housing 101 along a length direction of the water tank 103 is provided with two first fixing plates 113 that are spaced from each other, and two sidewalls of the bottom housing 101 along the width direction of the water tank 103 each are provided with a second fixing plate 114 that corresponds to the first fixing plates 113 and abuts against one side of the water tank 103. Third fixing plates 115 are provided between the two first fixing plates 113 and correspond to the second fixing plates 104. The third fixing plates 115 abut against another side of the water tank 103, thereby achieving the function of fixing the water tank 103.

In this embodiment, the two sidewalls of the bottom housing 101 along the width direction of the water tank 103 each are provided with a fourth fixing plate 116 that extends into the notch 1111 and abuts the heat sink 111, thereby achieving the function of fixing the heat sink 111.

In this embodiment, the cooling fan 112 is set obliquely between corresponding second fixing plate 114 and fourth fixing plate 116, with one side thereof abutting the second fixing plate 114 and another side thereof abutting the fourth fixing plate 116, thereby achieving the function of fixing the cooling fan 112. At the same time, since the two cooling fans 112 are set obliquely in the two notches 1111 of the two heat sinks 111, respectively, it is easier to quickly dissipate the heat of the heat sink 111 from one side close to the notch 111 to another side away from the notch 111.

In this embodiment, four sidewalls of the bottom housing 101 define a plurality of first vents 117 that extend vertically, for facilitating dissipation of heat from controller 100 to the outside.

In this embodiment, a bottom of the bottom housing 101 is provided with two rows of second vents 118 that are arranged in sequence and spaced from each other, for facilitating dissipation of heat from the controller 100 to the outside.

In this embodiment, a sidewall of the top housing 102 neighboring to the two first fixing plates 113 defines a plurality of third vents 1021 that extend transversely and correspond to the first vents 117, for facilitating dissipation of heat from the controller 100 to the outside.

In this embodiment, a water injection pipe 1061 is provided on the tank cover 10, and a mounting port 1022 is provided on the top housing 102 corresponding to the water injection pipe 1061. Further, a pipe cover 1023 is provided on the mounting port 1022.

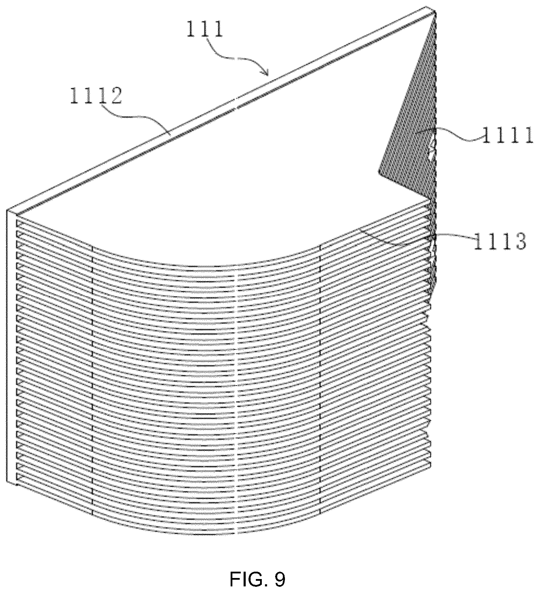

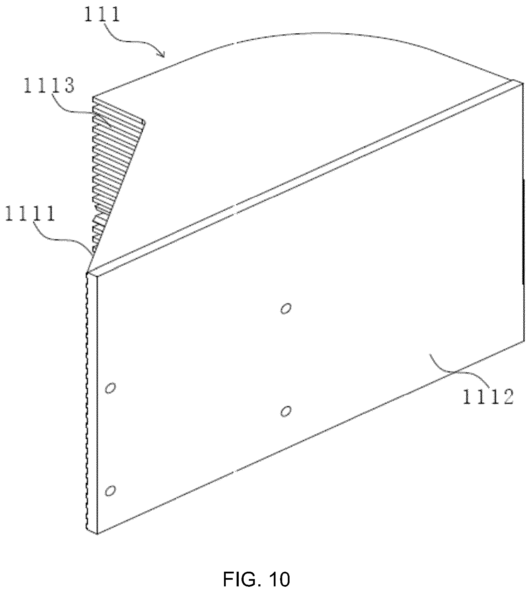

It should be noted that in this embodiment, as shown in FIG. 9 and FIG. 10, the heat sink 111 is preferably made of aluminum, which is convenient for heat dissipation. The heat sink 111 includes a substrate 1112 attached to the outer side of the cooling plate 110 and a plurality of cooling fins 1113 which are arranged on a side of the substrate 1112 away from the cooling plate 110 and spaced from each other. As the two cooling fans 112 are set obliquely in the notches 1111 of the two heat sinks 111, respectively, it is easier to quickly dissipate the heat of the heat sinks 111 from one side close to the notch 1111 to another side away from the notch 1111, facilitating the heat dissipation of the heat sinks 110.

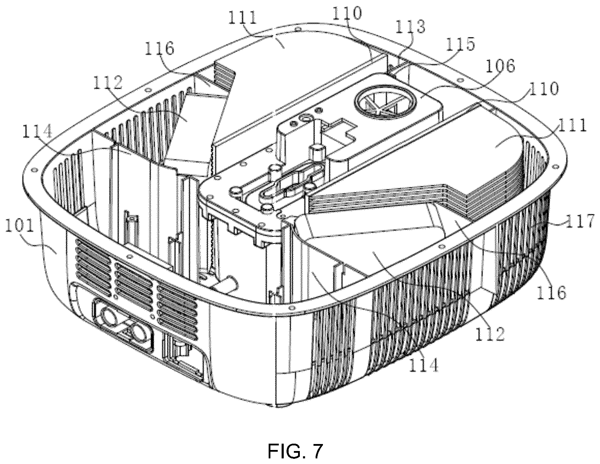

It should be noted that in this embodiment, as shown in FIG. 7 and FIG. 8, the heat exchangers 109 is preferably made aluminum, which is convenient for heat absorption and thus to cool the water in the water tank 103.

It should be noted that in this embodiment, a sidewall of the bottom housing 101 along its length direction is provided with a water pipe joint 119 corresponding to the outlet pipe and return pipe.

It should be noted that in this embodiment, a water injection pipe 1061 is provided on the tank cover 10, and a mounting port 1022 is provided on the top housing 102 corresponding to the water injection pipe 1061. Further, a pipe cover 1023 is provided on the mounting port 1022

The present application provides a controller for cool and warm dual system, which includes a bottom housing and the top housing, a water tank and a water pump connected to the water tank is provided in the bottom housing, a heater is provided in the water tank, a tank cover is arranged on the water tank, and a heating sealing gasket is sandwiched between the tank cover and the heater. Accommodating slots are defined in and extend through two sidewalls of the water tank along its width direction, respectively. The two accommodating slots each accommodate a heat exchanger that extends into the water tank. Outer sides of the two heat exchangers are respectively provided with a cooling plate, and outer sides of the two cooling plates are respectively provided with a semi-circular shaped heat sink. Opposite sides of the two heat sinks away from each other each define an inclined notch therein, and cooling fans matching with the notches are arranged in the two notches, respectively. The cooling fan is set obliquely, and attaches to the heat sink.

In this way, by setting of the heat exchangers, the cooling plates, the heat sinks and the cooling fans, when the mattress needs to be heated, the heater is started to heat the water in the water tank, which is delivered to the mattress under the action of the water pump and through the outlet pipe. The water that flows through the mattress is circulated to the water tank through the return pipe, thereby achieving the effect of circulating heating the mattress; when the mattress needs to be cooled, the two cooling plates are activated to absorb heat from the water of the water tank through the two heat exchangers, respectively, thereby achieving cooling of the water in the water tank, which is delivered to the mattress under the action of the water pump and through the outlet pipe. The water that flows through the mattress is circulated to the water tank through the return pipe, thereby achieving the effect of circulating cooling the mattress. At the same time, heat of the two cooling plates is transferred to the two heat sinks, respectively, and then dissipated to the outside through the two cooling fans along a direction from one side adjacent to the notch towards another side away from the notch. Thus, the present application can achieve the dual control functions of cooling and heating.

The above descriptions are only preferred embodiments of the present disclosure and are not intended to limit the present disclosure.

## Claims

1. A controller (100) for cool and warm dual system, the cool and warm dual system further comprising a mattress and outlet and return pipes connected between the controller (100) and the mattress, the controller (100) controlling the operation of the cool and warm dual system and **characterized in that** it comprising:
a bottom housing (101) and a top housing (102) a water tank (103) and a water pump (104) connected to the water tank (103) being provided in the bottom housing (101), a heater (105) being provided in the water tank (103), a tank cover (106) being provided on the water tank (103), and a heating sealing gasket (107) being sandwiched between the tank cover (106) and the heater (105),
accommodating slots (108) being defined in and extending through two sidewalls of the water tank (103) along its width direction, respectively, cooling end heat exchangers (109) being respectively accommodated in the accommodating slots (108) and extending into the water tank (103), outer sides of the cooling end heat exchangers (109) being respectively provided with a cooling plate (110), and outer sides of the cooling plates (110) being respectively provided with a semi-circular shaped heat sink (111),
opposite sides of the two heat sinks (111) respectively defining an inclined notch (1111) therein, and cooling fans (102) matching with the notches (1111) being respectively arranged in the notches (1111), the cooling fans (102) being set obliquely and attaching to the heat sinks (111), respectively.

2. The controller (100) for cool and warm dual system as claimed in claim 1, wherein a sidewall of the bottom housing (101) along a length direction of the water tank (103) is provided with two first fixing plates (113) that are spaced from each other, two sidewalls of the bottom housing (101) along the width direction of the water tank (103) each are provided with a second fixing plate (114) that corresponds to the first fixing plates (113) and abuts one side of the water tank (103), and third fixing plates (115) are provided between the two first fixing plates (113), correspond to the second fixing plates (114), and abut another side of the water tank (103).

3. The controller (100) for cool and warm dual system as claimed in claim 2, wherein two sidewalls of the bottom housing (101) along the width direction of the water tank (103) each are provided with a fourth fixing plate (116) that extends into the notch (1111) and abuts the heat sink (111).

4. The controller (100) for cool and warm dual system as claimed in claim 3, wherein the cooling fan (102) is set obliquely between corresponding second fixing plate (114) and fourth fixing plate (116), with one side thereof abutting the second fixing plate (114) and another side thereof abutting the fourth fixing plate (116).

5. The controller (100) for cool and warm dual system as claimed in claim 2, wherein four sidewalls of the bottom housing (101) define a plurality of first vents (117) that extend vertically, and a bottom of the bottom housing (101) is provided with two rows of second vents (118) that are arranged in sequence and spaced from each other.

6. The controller (100) for cool and warm dual system as claimed in claim 5, wherein a sidewall of the top housing (102) neighboring to the two first fixing plates (113) defines a plurality of third vents (1021) that extend transversely and correspond to the first vents (117).

7. The controller (100) for cool and warm dual system as claimed in claim 1, wherein a water injection pipe (1061) is provided on the tank cover (106), a mounting port (1022) is provided on the top housing (102) corresponding to the water injection pipe (1061), and a pipe cover (1023) is provided on the mounting port (1022).

## Patentansprüche

1. Steuerung (100) für ein Kühl- und Warm-Doppelsystem, wobei das Kühl- und Warm-Doppelsystem ferner eine Matratze und Auslass- und Rücklaufrohre umfasst, die zwischen der Steuerung (100) und der Matratze verbunden sind, wobei die Steuerung (100) den Betrieb des Kühl- und Warm-Doppelsystems steuert und **dadurch gekennzeichnet ist, dass** sie umfasst:
ein unteres Gehäuse (101) und ein oberes Gehäuse (102), einen Wassertank (103) und eine Wasserpumpe (104), die mit dem Wassertank (103) verbunden ist, die im unteren Gehäuse (101) vorgesehen sind, einen Heizer (105), der im Wassertank (103) vorgesehen ist, eine Tankabdeckung (106), die auf dem Wassertank (103) vorgesehen ist, und eine Heizungsdichtungsmanschette (107), die zwischen der Tankabdeckung (106) und dem Heizer (105) eingelegt ist,
Aufnahmeschlitze (108), die jeweils in zwei Seitenwänden des Wassertanks (103) definiert sind und sich durch diese entlang seiner Breitenrichtung erstrecken, Kühlungsendenwärmetauscher (109) jeweils in den Aufnahmeschlitzen (108) aufgenommen sind und sich in den Wassertank (103) hinein erstrecken, Außenseiten der Kühlungsendenwärmetauscher (109) jeweils mit einer Kühlungsplatte (110) versehen sind, und Außenseiten der Kühlungsplatten (110) jeweils mit einer halbkreisförmigen Wärmesenke (111) versehen sind,
entgegengesetzte Seiten der zwei Wärmesenken (111) jeweils eine geneigte Aussparung (1111) darin definieren, und Kühlgebläsen (102), die zu den Aussparungen (1111) passen, jeweils in den Aussparungen (1111) angeordnet sind, wobei die Kühlgebläsen (102) jeweils schräg gesetzt sind und sich an die Wärmesenken (111) anschließen.

2. Steuerung (100) für ein Kühl- und Warm-Doppelsystem nach Anspruch 1, worin eine Seitenwand des unteren Gehäuses (101) entlang einer Längenrichtung des Wassertanks (103) mit zwei ersten Fixierplatten (113) versehen ist, die voneinander beabstandet sind, zwei Seitenwände des unteren Gehäuses (101) entlang der Breitenrichtung des Wassertanks (103) je mit einer zweiten Fixierplatte (114) versehen sind, die den ersten Fixierplatten (113) entsprechen und an eine Seite des Wassertanks (103) anliegen, und dritte Fixierplatten (115) zwischen den zwei ersten Fixierplatten (113) vorgesehen sind, den zweiten Fixierplatten (114) entsprechen und an eine andere Seite des Wassertanks (103) anliegen.

3. Steuerung (100) für ein Kühl- und Warm-Doppelsystem nach Anspruch 2, worin zwei Seitenwände des unteren Gehäuses (101) entlang der Breitenrichtung des Wassertanks (103) je mit einer vierten Fixierplatte (116) versehen sind, die sich in die Aussparung (1111) hinein erstreckt und an die Wärmesenke (111) anliegt.

4. Steuerung (100) für ein Kühl- und Warm-Doppelsystem nach Anspruch 3, worin das Kühlgebläse (102) schräg zwischen der entsprechenden zweiten Fixierplatte (114) und der vierten Fixierplatte (116) gesetzt ist, mit einer Seite davon, die an die zweite Fixierplatte (114) anliegt, und einer anderen Seite davon, die an die vierte Fixierplatte (116) anliegt.

5. Steuerung (100) für ein Kühl- und Warm-Doppelsystem nach Anspruch 2, worin vier Seitenwände des unteren Gehäuses (101) eine Vielzahl von ersten Lüftungsöffnungen (117) definieren, die sich vertikal erstrecken, und ein Boden des unteren Gehäuses (101) mit zwei Reihen von zweiten Lüftungsöffnungen (118) versehen ist, die nacheinander angeordnet sind und voneinander beabstandet sind.

6. Steuerung (100) für ein Kühl- und Warm-Doppelsystem nach Anspruch 5, worin eine Seitenwand des oberen Gehäuses (102), die den zwei ersten Fixierplatten (113) benachbart ist, eine Vielzahl von dritten Lüftungsöffnungen (1021) definiert, die sich quer erstrecken und den ersten Lüftungsöffnungen (117) entsprechen.

7. Steuerung (100) für ein Kühl- und Warm-Doppelsystem nach Anspruch 1, worin ein Wassereinspritzrohr (1061) an der Tankabdeckung (106) vorgesehen ist, ein Montageanschluss (1022) am oberen Gehäuse (102) dem Wassereinspritzrohr (1061) entsprechend vorgesehen ist, und eine Rohrabdeckung (1023) am Montageanschluss (1022) vorgesehen ist.

## Revendications

1. Dispositif de commande (100) pour système double froid et chaud, le système double froid et chaud comprenant en outre un matelas et des tuyaux de sortie et de retour connectés entre le dispositif de commande (100) et le matelas, le dispositif de commande (100) commandant le fonctionnement du système double froid et chaud et **caractérisé en ce qu'**il comprend :
un boîtier inférieur (101) et un boîtier supérieur (102), un réservoir d'eau (103) et une pompe à eau (104) reliés au réservoir d'eau (103) étant prévus dans le boîtier inférieur (101), un dispositif de chauffage (105) étant prévu dans le réservoir d'eau (103), un couvercle de réservoir (106) étant prévu sur le réservoir d'eau (103), et un joint d'étanchéité de chauffage (107) étant pris en sandwich entre le couvercle de réservoir (106) et le dispositif de chauffage (105),
des fentes de réception (108) étant définies dans et s'étendant à travers deux parois latérales du réservoir d'eau (103) le long de sa direction de largeur, respectivement, des échangeurs de chaleur d'extrémité de refroidissement (109) étant respectivement reçus dans les fentes de réception (108) et s'étendant dans le réservoir d'eau (103), les côtés extérieurs des échangeurs de chaleur d'extrémité de refroidissement (109) étant respectivement pourvus d'une plaque de refroidissement (110), et les côtés extérieurs des plaques de refroidissement (110) étant respectivement pourvus d'un dissipateur thermique de forme semi-circulaire (111),
les côtés opposés des deux dissipateurs thermiques (111) définissant respectivement une encoche inclinée (1111) dans ceux-ci, et des ventilateurs de refroidissement (102) correspondant aux encoches (1111) étant respectivement disposés dans les encoches (1111), les ventilateurs de refroidissement (102) étant disposés obliquement et se fixant aux dissipateurs thermiques (111), respectivement.

2. Dispositif de commande (100) pour système double froid et chaud selon la revendication 1, dans lequel une paroi latérale du boîtier inférieur (101) le long d'une direction de longueur du réservoir d'eau (103) est pourvue de deux premières plaques de fixation (113) qui sont espacées l'une de l'autre, deux parois latérales du boîtier inférieur (101) le long de la direction de largeur du réservoir d'eau (103) sont chacune pourvues d'une deuxième plaque de fixation (114) qui correspond aux premières plaques de fixation (113) et vient en butée contre un côté du réservoir d'eau (103), et des troisièmes plaques de fixation (115) sont prévues entre les deux premières plaques de fixation (113), correspondent aux deuxièmes plaques de fixation (114) et viennent en butée contre un autre côté du réservoir d'eau (103).

3. Dispositif de commande (100) pour système double froid et chaud selon la revendication 2, dans lequel deux parois latérales du boîtier inférieur (101) le long de la direction de la largeur du réservoir d'eau (103) sont chacune pourvues d'une quatrième plaque de fixation (116) qui s'étend dans l'encoche (1111) et vient en butée contre le dissipateur thermique (111).

4. Dispositif de commande (100) pour système double froid et chaud selon la revendication 3, dans lequel le ventilateur de refroidissement (102) est réglé obliquement entre la deuxième plaque de fixation (114) correspondante et la quatrième plaque de fixation (116), avec un côté de celui-ci en butée contre la deuxième plaque de fixation (114) et un autre côté de celui-ci en butée contre la quatrième plaque de fixation (116).

5. Dispositif de commande (100) pour système double froid et chaud selon la revendication 2, dans lequel quatre parois latérales du boîtier inférieur (101) définissent une pluralité de premiers évents (117) qui s'étendent verticalement, et un fond du boîtier inférieur (101) est pourvu de deux rangées de deuxièmes évents (118) qui sont disposés en séquence et espacés les uns des autres.

6. Dispositif de commande (100) pour système double froid et chaud selon la revendication 5, dans lequel une paroi latérale du boîtier supérieur (102) voisine des deux premières plaques de fixation (113) définit une pluralité de troisièmes évents (1021) qui s'étendent transversalement et correspondent aux premiers évents (117).

7. Dispositif de commande (100) pour système double froid et chaud selon la revendication 1, dans lequel un tuyau d'injection d'eau (1061) est prévu sur le couvercle de réservoir (106), un orifice de montage (1022) est prévu sur le boîtier supérieur (102) correspondant au tuyau d'injection d'eau (1061), et un couvercle de tuyau (1023) est prévu sur l'orifice de montage (1022).
